# EUROPÄISCHE PATENTSCHRIFT

(11) **EP 0 708 758 B1**
(45) Veröffentlichungstag und Bekanntmachung des Hinweises auf die Patenterteilung: **04.02.1998**
(21) Anmeldenummer: 94924727.4
(22) Anmeldetag: 08.07.1994
(51) Int. Cl.: C07D 239/36, C07C 229/30

(54) **VERFAHREN ZUR HERSTELLUNG VON 5-CHLOR-4-HYDROXYPYRIMIDINEN, 2-CHLORENAMINE ALS ZWISCHENPRODUKTE DIESES VERFAHRENS UND DEREN VERWENDUNG**
METHOD OF PREPARING 5-CHLORO-4-HYDROXYPYRIMIDINES, 2-CHLORENAMINES AS INTERMEDIATES IN THE METHOD, AND USE OF THE COMPOUNDS THUS PREPARED
PROCEDE DE FABRICATION DE 5-CHLORO-4-HYDROXYPYRIMIDINES, 2-CHLORENE-AMINE EN TANT QUE PRODUITS INTERMEDIAIRES DE CE PROCEDE ET UTILISATION DE CES COMPOSES

(30) Priorität: 10.07.1993 DE 4323180
(43) Veröffentlichungstag der Anmeldung: 01.05.1996
(73) Patentinhaber: Hoechst Schering AgrEvo GmbH, 13509 Berlin (DE)
(72) Erfinder: ZURMÜHLEN, Frank, D-65929 Frankfurt am Main (DE)
(86) Internationale Anmeldenummer: EP9402244
(87) Internationale Veröffentlichungsnummer: WO9501963

(56) Entgegenhaltungen:
- EP-A- 0 326 389
- EP-A- 0 370 391
- EP-A- 0 568 041
- US-A- 4 199 600

## Beschreibung

5-Chlor-4-hydroxypyrimidine sind wichtige Zwischenprodukte zur Herstellung von Pflanzenschutz - und Arzneimitteln, wie sie beispielsweise in der PCT/EP 93/00536 vorgeschlagen werden.

5-Chlor-4-hydroxypyrimidine werden auch als 5-Chlor-4-pyrimidone oder als 5-Chlor-4-pyrimidinole bezeichnet. Sie werden im allgemeinen durch Chlorierung von 4-Hydroxypyrimidinen in 5-Stellung hergestellt. Als Chlorierungsmittel werden dabei beispielsweise N-Chlorsuccinimid, Natriumhypochlorit, Thionylchlorid [D. J. Brown, The Chemistry of Heterocyclic Compounds, The Pyrimidines (1962); ibid.; The Pyrimidines Supplement I (1970); ibid., The Pyrimidines Supplement II (1985); alle erschienen bei John Wiley & Sons Inc., New York] oder Chlorgas [JP 8222070] verwendet. Dabei wirken sich die häufig schlechten Ausbeuten ungünstig für einen technischen Prozeß aus. Nachteilig ist auch, daß die als Ausgangsstoffe erforderlichen 4-Hydroxypyrimidine nur schlecht zugänglich sind.

Auf die bisher bekannten Verfahren, die zu 4-Hydroxypyrimidinen führen, soll im folgenden näher eingegangen werden.

Im ersten Fall wird ein β-Ketosäureester mit Thioharnstoff zu dem entsprechenden 2-Thiouracil kondensiert und anschließend mit Raney-Nickel entschwefelt [H. M. Foster, H. R. Snyder, Org. Synth., Coll. Vol. IV, 638]. Diese Entschwefelung ist jedoch für einen technischen Prozeß nicht praktikabel.

Als weiteres Verfahren zur Herstellung von 4-Hydroxypyrimidinen ist die Kondensationsreaktion eines β-Ketosäureesters mit Formamidinacetat beschrieben [M.Butters, J. Heterocycl. Chem., 29, 1369 (1992)]. Die Ausbeute dieser Reaktion ist jedoch sehr niedrig und der Salzanfall relativ hoch; auch ist Formamidinacetat ein relativ teures Kondensationsmittel, so daß dieses Verfahren keine technische Alternative darstellt.

Eine weitere Herstellungsmöglichkeit für 4-Hydroxypyrimidine ist die Umsetzung eines aus einem β-Ketoester dargestellten Enamins mit Formamid [EP-A-0326389]. Diese Synthese ist an sich für ein technisches Verfahren geeignet, besitzt jedoch den Nachteil, daß sich in 5-Stellung unsubstituierte 4-Hydroxypyrimidine, insbesondere mit kurzkettigen Alkylresten, bedingt durch ihre hohe Wasserlöslichkeit wesentlich schwerer aufarbeiten lassen als die entsprechenden in 5-Stellung chlorsubstituierten Vertreter. Aufgrund dieser Aufarbeitungsverluste sowie durch die schon erwähnten Verluste bei der anschließenden 5-Chlorierung ist es nachteilig diesen Weg im Sinne einer technischen Synthese zu beschreiten.

Es ist dagegen bedeutend günstiger, eine Kondensationsreaktion durchzuführen bei der direkt ein 5-Chlor-4-hydroxypyrimidin gebildet wird.

Ein solches Verfahren, bei dem 2-Chlor-β-ketosäureester mit Formamidinsalzen direkt zu 5-Chlor-4-hydroxypyrimidinen umgesetzt werden, ist bereits beschrieben [EP-A-0370391]. Dieses Verfahren weist jedoch den Nachteil auf, daß es mit dem Einsatz von Formamidinacetat auf ein relativ teures Kondensationsmittel angewiesen ist. Daneben ist die Abfallsituation nicht unproblematisch; durch die ungünstige Stöchiometrie wird in Kauf genommen, daß der im Überschuß eingesetzte Teil des Formamidinacetats polymerisiert und daher nicht zurückgewonnen werden kann. Darüberhinaus ist zur Freisetzung von Formamidin aus seinem Salz ein hoher Basenüberschuß notwendig, was mit einer hohen Salzfracht einhergeht.

Aufgabe der Erfindung war es daher ein Verfahren zur Herstellung von 5-Chlor-4-hydroxypyrimidinen zu finden, das im technischen Maßstab mit einem preiswerten Kondensationsmittel durchgeführt werden kann und eine günstige Umweltbilanz (= geringer Salzanfall) aufweist.

Die erfindungsgemäße Aufgabe wird überraschenderweise dadurch gelöst, daß ein 2-Chlor-β-ketoester mit Ammoniak oder einem Ammoniumsalz in das entsprechende Enamin überführt wird, welches anschließend mit Formamid zum 5-Chlor-4-hydroxypyrimidin kondensiert wird. Von den dabei als Zwischenprodukte verwendeten Enaminen sind bereits 3-Amino-2-chlorcrotonsäure-(C₁-C₄)-alkylester aus US-Patent 4199600 bekannt.

Die Erfindung betrifft daher ein Verfahren zur Herstellung von 5-Chlor-4-hydroxypyrimidin der allgemeinen Formel I 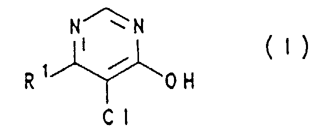 das dadurch gekennzeichnet ist, daß man einen 2-Chlor-β-ketoester der allgemeinen Formel II 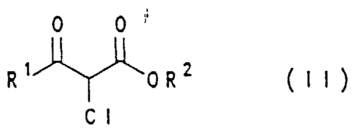 mit Ammoniak oder dem Ammoniumsalz vorzugsweise einer organischen Säure zu einem 2-Chlorenamin der allgemeinen Formel III 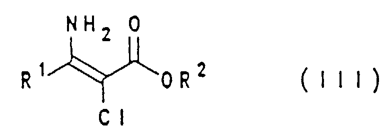 umgesetzt wird (die Umsetzung kann in polar protischen oder aprotischen Lösungsmitteln oder ohne Solventien durchgeführt werden) und das anschließend in einem polar protischen Solvens in Gegenwart einer Base mit Formamid zur Verbindung der allgemeinen Formel I kondensiert.

Das erfindungsgemäße Verfahren wird vorzugsweise ohne Isolierung des 2-Chlorenamins der Formel III (als "Eintopfreaktion") durchgeführt.

Es besteht dann beispielsweise die Möglichkeit, das entstehende Produkt der Formel I mit POCl₃ in an sich bekannter Weise zu einem 4,5-Dichlorpyrimidin der allgemeinen Formel IV 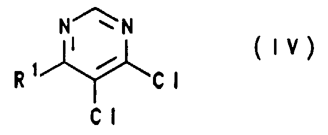 umzusetzen.

In den Formeln I - IV bedeuten
- R¹: gegebenenfalls substituiertes Alkyl, Cycloalkyl, Aryl oder Benzyl,
- R²: Alkyl, Benzyl oder eine andere Carboxylschutzgruppe.
- R¹: bedeutet vorzugsweise (C₁-C₄)-Alkyl, (C₁-C₄)-Halogenalkyl, (C₁-C₄)-Alkoxy-(C₁-C₄)-alkyl oder (C₁-C₄)-Alkylthio-(C₁-C₄)-alkyl, insbesondere (C₁-C₄)-Alkyl, wie Methyl oder Ethyl, oder Methoxymethyl; ganz besonders bevorzugt ist Ethyl.
- R²: bedeutet vorzugsweise (C₁-C₄)-Alkyl, Benzyl oder eine andere Carboxylschutzgruppe, insbesondere (C₁-C₄)-Alkyl, wie Methyl, Ethyl oder tert.-Butyl, Benzyl oder modifiziertes Benzyl; ganz besonders bevorzugt ist Methyl.

Falls im einzelnen nicht anders definiert, ist Alkyl geradkettig oder verzweigt und steht vorzugsweise für (C₁-C₆)-Alkyl, wie Methyl, Ethyl, Propyl, Isopropyl, Butyl, tert.-Butyl, Pentyl, Hexyl. Entsprechendes gilt für davon abgeleitete Reste wie Alkoxy, Alkylthio, Halogenalkyl.

Cycloalkyl weist vorzugsweise 3 bis 8 C-Atome auf und steht für Reste wie Cyclobutyl, Cyclopentyl, Cyclohexyl.

Aryl weist vorzugsweise 6 bis 12 C-Atome auf und steht beispielsweise für Phenyl, Naphthyl, Biphenylyl; bevorzugt ist Phenyl.

Substituiertes Alkyl, Cycloalkyl, Aryl oder Benzyl ist vorzugsweise mit 1 bis 3 gleichen oder verschiedenen Resten substituiert, die sich unter den Bedingungen des erfindungsgemäßen Verfahrens inert verhalten und ausgewählt sind aus Halogen, (C₁-C₄)-Alkoxy, (C₁-C₄)-Alkylthio, und, im Falle der cyclischen Reste, (C₁-C₄)-Alkyl.

Unter Halogen versteht man Fluor, Chlor, Brom oder lod, vorzugsweise Fluor oder Chlor.

Halogenalkyl ist ein wie oben definierter Alkylrest, in welchem ein, mehrere oder alle Wasserstoffatome durch gleiche oder verschiedene Halogenatome substituiert sind.

Carboxylschutzgruppen werden z.B. in Hubbuch, Kontakte Merck 3/79, Seiten 14 und 19 ff. beschrieben. Häufig verwendet werden Methyl, Ethyl, Benzyl und tert.-Butyl, sowie modifizierte Benzylreste, wie p-Chlorbenzyl, p-Nitrobenzyl, p-Methoxybenzyl.

Die Erfindung betrifft auch 2-Chlorenamine der Formel III, in welcher R¹ und R² wie oben definiert sind, ausgenommen 3-Amino-2-chlor-crotonsäure-(C₁-C₄)-alkylester.

Die in Formel II dargestellten 2-Chlor-β-ketoester können in bekannter Weise durch Chiorierung der entsprechenden β-Ketoester mit Sulfurylchlorid wahlweise unter Verwendung eines aprotischen Solvens oder lösemittelfrei [W. R. Böhme, Org. Synth. Coll. Vol. IV, 590 (1963)] sowie mit Chlorgas als Chlorierungsmittel hergestellt werden.

Zur Herstellung des 2-Chlorenamins der Formel III kann das Rohprodukt aus Stufe 1 nach Abtrennung des Lösemittels und der Gase eingesetzt werden. Die Umsetzung kann mit NH₃-Gas in Substanz, in polar protischen Lösemitteln wie Alkoholen oder Formamid oder in polar aprotischen Solventien bspw. Dioxan , Dimethylformamid oder Acetonitril durchgeführt werden. Anstelle von NH₃ können auch Ammoniumsalze als Ammoniaklieferanten eingesetzt werden. Als Alkohole finden niedere Alkohole wie Methanol, Ethanol, Isopropanol oder Butanol Verwendung. Bei der Verwendung von Ammoniumsalzen wirkt sich ein Zusatz von Polyethylenglykol ausbeutesteigernd aus.

Die Umsetzung kann in einem Temperaturbereich von -40 bis 80 °C , bevorzugt von 20 bis 78 °C durchgeführt werden. Als Ammoniumsalze finden bevorzugt Ammoniumsalze der Carbonsäuren wie Ameisensäure, Essigsäure, Oxalsäure oder der Kohlensäure Verwendung. Die Aufarbeitung erfolgt bevorzugt wasserfrei, indem nach Entfernen des Solvens im Vakuum die Substanz in einem aprotischen Solvens wie Diisopropylether oder Methylisobutylketon gelöst wird und vom Überschuß Ammoniumsalz abfiltriert wird. Das zurückgewonnene Ammoniumsalz kann erneut eingesetzt werden.

Für die Kondensationsreaktion werden bevorzugt polar protische Lösungsmittel wie Wasser, Methanol, Ethanol, i-Propanol oder Butanol eingesetzt. Bevorzugt ist die Verwendung desjenigen Alkohols der mit dem entsprechenden Ester korrespondiert, d. h. Methanol oder Ethanol. Bei einer azeotropen Fahrweise ist es von Vorteil die benötigte Menge eines Schleppers beispielsweise Toluol zuzusetzen.

Als Basen kommen Alkalialkoholate, -hydroxide, -carbonate oder -hydrogencarbonate zum Einsatz. Bevorzugt ist die Fahrweise mit einem Alkalialkoholat in dem entsprechenden Alkohol. Hierbei kann gegebenenfalls ein Trockenmittel, beispielsweise Magnesiumsulfat oder Molekularsieb 30 nm, zum Binden des bei der Reaktion freiwerdenden Wassers zugesetzt werden. Die Reaktionstemperatur liegt zwischen 20 und 80 °C. Vorzugsweise wird ein Überschuß an Formamid von 1,5 - 3,5 Mol des theoretisch erforderlichen eingesetzt. Ein Teil des Formamidüberschuß wird bei der Reaktion nicht verbraucht und kann nach Destillation wieder verwendet werden. Für 1 Mol eingesetztem 2-Chlorenamin der Formel III sind 1 - 2,2 Mol Base notwendig. Nach beendeter Reaktion kann das hergestellte 5-Chlor-4-hydroxypyrimidin der Formel II nach Lösen in Wasser durch Extraktion bei einem pH von 3 - 7 mit einem polaren Lösungsmittel extrahiert werden. Bei einigen Derivaten kristallisiert das 5-Chlorpyrimidin beim angegebenen pH-Wert aus Wasser aus, so daß es anschließend abgesaugt werden kann.

Es ist aber auch möglich, daß Rohprodukt nach dem Ansäuern auf pH 3 - 7 und Entfernen des Lösungsmittels im Vakuum ohne weitere Aufreinigung mit POCl₃ zum entsprechenden 4,5-Dichlorpyrimidin umzusetzen.

Die nachfolgenden Beispiele illustrieren die Erfindung, ohne daß diese darauf beschränkt wäre.

### Beispiel 1

### 3-Amino-2-chlorpent-2-ensäuremethylester

Durch 48,9 g (0,30 Mol) 2-Chlor-3-oxovaleriansäuremethylester wird bei 70 °C während eines Zeitraumes von 2 h trockenes NH₃-Gas geleitet. Nach Entfernen der flüchtigen Bestandteile im Vakuum wird das Rohprodukt in Diisopropylether gelöst und mit wenig Eiswasser gewaschen. Nach Trocknen über MgSO₄ wird das Lösungsmittel im Vakuum entfernt.
Ausbeute: 80,0 % [GC]

### Beispiel 2

### 3-Amino-2-chlorpent-2-ensäuremethylester

Man erhitzt eine Lösung von 576,1 g (3,5 Mol) 2-Chlor-3-oxovaleriansäuremethylester und 674,5 g (8,75 Mol) Ammoniumacetat in 1,2 l Methanol unter Rühren während eines Zeitraumes von 4 h auf 65 °C. Nach dem Abkühlen wird das Lösungsmittel im Vakuum entfernt, der Rückstand in 2,5 l Diisopropylether aufgenommen und vom Feststoff abgetrennt. Der Feststoff wird in wenig Wasser gelöst und anschließend mit Essigester extrahiert. Die organischen Fraktionen werden vereinigt, über MgSO₄ getrocknet und im Vakuum eingedampft.
Ausbeute: 94,7 % [GC]

### Beispiel 3

### 3-Amino-2-chlorpent-2-ensäuremethylester

Eine Lösung von 47,5 g (0,75 Mol) Ammoniumformiat und 49,0 g (0,3 Mol) 2-Chlor-3-oxovaleriansäuremethylester in 800 ml Ethanol werden analog Beispiel 2 umgesetzt und aufgearbeitet.
Ausbeute: 72,0 % [GC]

### Beispiel 4

### 3-Amino-2-chlorpent-2-ensäuremethylester

Durch eine Lösung von 376,3 g (2,064 Mol) 2-Chlor-3-oxovaleriansäuremethylester in 100 ml Methanol leitet man bei 65 °C unter Rühren innerhalb von 3 h trockenes NH₃-Gas. Das Lösungsmittel wird im Vakuum entfernt, der Rückstand in Diisopropylether aufgenommen, über Seesand filtriert und das Lösungsmittel eingedampft.
Ausbeute: 87,6 % [GC]

### Beispiel 5

### 3-Amino-2-chlorbut-2-ensäuremethylester

50 g (0,33 Mol) 2-Chloracetessigsäuremethylester werden mit 64 g (0,83 Mol) Ammoniumacetat in 100 ml Methanol analog Beispiel 2 umgesetzt und aufgearbeitet.
Ausbeute: 63,9 % [GC]

### Beispiel 6

### 3-Amino-2-chlorhex-2-ensäureethylester

61,5 g (0,32 Mol) 2-Chor-3-oxohexansäureethylester werden mit 61,5 g (0,80 Mol) Ammoniumacetat in 120 ml Methanol analog Beispiel 2 umgesetzt und aufgearbeitet.
Ausbeute: 95,5 % [GC]

### Beispiel 7

### 3-Amino-2-chlor-4-methylpent-2-ensäureethylester

59,5 g (0,31 Mol) 2-Chlor-4-methyl-3-oxopentansäureethylester werden analog Beispiel 2 mit 59,8 g (0,78 Mol) Ammoniumacetat in 120 ml Methanol umgesetzt und aufgearbeitet.
Ausbeute: 88,0 % [GC]

### Beispiel 8

### 3-Amino-2-chlor-3-phenylpropensäureethylester

58,25 g (0,26 Mol) 2-Chlorbenzoylessigsäureethylester werden mit 49,52 g (0,64 Mol) Ammoniumacetat in 100 ml Methanol analog zu Beispiel 2 umgesetzt und aufgearbeitet.
Ausbeute: 91,6 % [GC]

### Beispiel 9

### 5-Chlor-6-ethyl-4-hydroxypyrimidin

Man tropft eine Mischung von 163,6 g (1,0 Mol) 3-Amino-2-chlorpent-2-ensäuremethylester, 90,8 g (2,0 Mol) Formamid und 150 ml Methanol unter Rühren bei Raumtemperatur zu 485,8 ml einer 30 %igen NaOMe-Lösung in Methanol. Anschließend heizt man langsam innerhalb von 3 h bis zur Rückflußtemperatur und läßt bei dieser Temperatur noch weitere 12 h rühren. Nach Eindampfen des Reaktionsgemisches löst man in wenig H₂O stellt mit HCl einen pH-Wert von 3,8 ein und extrahiert mit Essigester. Trocknen über MgSO₄ und Eindampfen liefert 177,5 g Rohprodukt, das anschließend aus Wasser umkristallisiert wird.
Ausbeute: 130,4 g (82 %)

### Beispiel 10

### 5-Chlor-6-ethyl-4-hydroxypyrimidin

Zu 90,3 ml einer 30 %igen Natriummethylatlösung in Methanol wird unter Rühren bei Raumtemperatur gleichzeitig, unter Verwendung zweier Tropftrichter, eine Lösung von 35,5 g (0,2 Mol) 3-Amino-2-chlorpent-2-ensäuremethylester und 31,5 g (0,7 Mol) Formamid in 150 ml Methanol getropft. Man heizt langsam bis zur Rückflußtemperatur und beläßt dort 10 h. Nach Abkühlen des Ansatzes leitet man trockenes HCI-Gas bis zum pH 3 durch die Lösung. Nach Eindampfen des Lösungsmittels extrahiert man mit Butanon, trocknet und rotiert ein.
Ausbeute: 87 % [HPLC]

### Beispiel 11

### 5-Chlor-4-hydroxy-6-methylpyrimidin

25,9 g (0,17 Mol) 3-Amino-2-chlorbut-2-ensäuremethylester werden analog Beispiel 9 mit 11,7 g (0,26 Mol) Formamid und 51 ml Natriummethylatlösung in 20 ml Methanol umgesetzt und aufgearbeitet (Reaktionszeit 20 h).
Ausbeute: 16,6 g (67,5 %)

### Beispiel 12

### 5-Chlor-4-hydroxy-6-propylpyrimidin

58,1 g (0,30 Mol) 3-Amino-2-chlorhex-2-ensäureethylester werden analog Beispiel 9 mit 47,7 g (1,1 Mol) Formamid und 140 ml Natriummethylatlösung in 175 ml Methanol umgesetzt und aufgearbeitet (Reaktionszeit 6 h).
Ausbeute: 42,2 g (81,5 %)

### Beispiel 13

### 5-Chlor-4-hydroxy-6-phenylpyrimidin

45,73 g (0,20 Mol) 3-Amino-2-chlor-3-phenylpropensäureethylester werden analog Beispiel 9 mit 31,9 g (0,71 Mol) Formamid und 93 ml Natriummethylatlösung in 120 ml Methanol umgesetzt und aufgearbeitet (Reaktionszeit 16 h).
Ausbeute: 32,9 g (79,7 %),

### Beispiel 14

### 5-Chlor-4-hydroxy-6-isopropylpyrimidin

45,1 g (0,24 Mol) 3-Amino-2-chlor-4-methylpent-2-ensäureethylester wird analog Beispiel 9 mit 36,8 g (0,59 Mol) Formamid und 109 ml Natriummethylatlösung in 130 ml Methanol umgesetzt und aufgearbeitet.
Ausbeute: 34,5 g (83,3 %)

### Beispiel 15

### 5-Chlor-4-hydroxy-6-ethylpyrimidin (Eintopfverfahren)

134,0 g (0,82 Mol) 3-Amino-2-chlorpent-2-ensäuremethylester und 125,3 g Formamid, gelöst in 150 ml Methanol werden gleichzeitig unter Rühren bei Raumtemperatur zu 357,8 ml einer 30 %igen Natriummethylatlösung in Methanol getropft. Man heizt innerhalb von 5 h zur Rückflußtemperatur und beläßt dort noch 10 h. Nach dem Abkühlen wird solange trockenes HCI-Gas durchgeleitet, bis ein pH-Wert von 3 erreicht ist. Das Lösungsmittel und der Formamid Überschuß werden abdestilliert und anschließend zweimal 100 ml Toluol zugefügt und abdestilliert. Die Mischung wird mit 145,3 ml POCl₃ versetzt und 5 h unter Rühren auf 70-80 °C erhitzt. Anschließend wird der POCl₃ Überschuß abdestilliert, auf Eiswasser gegeben und mit K₂CO₃ neutralisiert. Nach Extraktion mit Essigester und Eindampfen des Lösungsmittels wird der Rückstand bei 110-112 °C bei 25 mbar destilliert.
Ausbeute: 114,0 g (78.5 %)

### Beispiel 16

### 5-Chlor-6-ethyl-4-hydroxypyrimidin

Durch eine Lösung von 99,7 g (0,6 Mol) 2-Chlor-3-oxovaleriansäuremethylester in 130 ml Formamid wird unter Kühlung (max. 50°C) NH₃-Gas geleitet, bis der Ansatz kein NH₃ mehr aufnimmt (ca. 1 h). Die Ammoniak-Reste werden durch Ausblasen mit Stickstoff und Anlegen von Vakuum entfernt. Anschließend tropft man die Reaktionslösung zu 450 ml einer 30 % NaOMe-Lösung in Methanol bei 50°C zu. Nach Zugabe von weiteren 50 ml Formamid erhitzt man 3h auf 50°C. Die flüchtigen Anteile werden im Vakuum abgedampft, anschließend wird auf Wasser gegeben und mit HCI auf pH 6 gestellt. Extraktion mit Ethylacetat und Eindampfen liefert 91,2 g Rohprodukt, das aus kaltem Aceton umkristallisiert wird.
Ausbeute: 66,3 g (70 %)

### Beispiel 17

### 5-Chlor-6-ethyl-4-hydroxypyrimidin

Durch eine Lösung von 150 g (0,88 Mol) 2-Chlor-3-oxovaleriansäuremethylester in 100 ml Methanol wird unter Kühlung (Reaktionstemperatur max. 50°C) trockenes NH₃-Gas geleitet, bis der Ansatz kein NH₃ mehr aufnimmt (2,5 h). Die Ammoniak-Reste werden durch Ausblasen mit N₂ und kurzes Anlagen eines Vakuum entfernt. Anschließend gibt man 140 ml Formamid zu und tropft die Lösung zu 472 ml einer 30 % NaOMe-Lösung in Methanol bei 50°C zu. Nach 4 h Erhitzen auf 50°C arbeitet man analog Beispiel 16 auf.
Ausbeute: 114,4 g (82 %)

## Patentansprüche

1. Verfahren zur Herstellung einer Verbindung der allgemeinen Formel I, 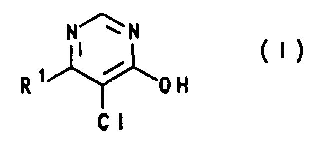 in welcher R¹ Alkyl, Cycloalkyl, Aryl oder Benzyl bedeutet, die alle gegebenenfalls substituiert sind, dadurch gekennzeichnet, daß man eine Verbindung der allgemeinen Formel II, 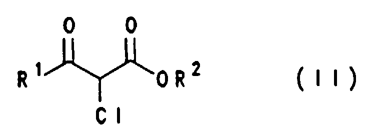 in welcher R¹ wie oben definiert ist und R² Alkyl, Benzyl oder eine andere Carboxylschutzgruppe bedeutet, mit Ammoniak oder einem Ammoniumsalz zu einer Verbindung der allgemeinen Formel III, 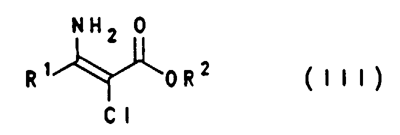 umsetzt, welches man anschließend in einem polar protischen Solvens in Gegenwart einer Base mit Formamid zu einer Verbindung der allgemeinen Formel I kondensiert.

2. Verfahren gemäß Anspruch 1, dadurch gekennzeichnet, daß ohne Isolierung des Zwischenprodukts der Formel III gearbeitet wird.

3. Verfahren gemäß Anspruch 1 oder 2, dadurch gekennzeichnet, daß Verbindungen der Formel I hergestellt werden, in welcher R¹ (C₁-C₄)-Alkyl, (C₁-C₄)-Halogenalkyl, (C₁-C₄)-Alkoxy-(C₁-C₄)-alkyl oder (C₁-C₄)-Alkylthio-(C₁-C₄)-alkyl bedeutet.

4. Verfahren gemäß einem der Ansprüche 1 bis 3, dadurch gekennzeichnet, daß Verbindungen der Formel I hergestellt werden, in welcher R¹ (C₁-C₄)-Alkyl, vorzugsweise Methyl oder Ethyl oder Methoxymethyl bedeutet.

5. Verfahren gemäß einem der Ansprüche 1 bis 4, dadurch gekennzeichnet, daß Verbindungen der Formel I hergestellt werden, in welcher R¹ Ethyl bedeutet.

6. Verfahren gemäß einem der Ansprüche 1 bis 5, dadurch gekennzeichnet, daß man von Verbindungen der Formel II ausgeht, in welcher R² (C₁-C₄)-Alkyl, Benzyl oder eine andere Carboxylschutzgruppe bedeutet.

7. Verfahren gemäß einem der Ansprüche 1 bis 6, dadurch gekennzeichnet, daß man von Verbindungen der Formel II ausgeht, in welcher R² (C₁-C₄)-Alkyl, vorzugsweise Methyl, Ethyl oder tert.-Butyl, Benzyl oder modifiziertes Benzyl bedeutet.

8. Verfahren gemäß einem der Ansprüche 1 bis 7, dadurch gekennzeichnet, daß man von Verbindungen der Formel II ausgeht, in welcher R² Methyl bedeutet.

9. Verbindung der Formel III gemäß Anspruch 1, in welcher R¹ und R² wie in einem der vorangehenden Ansprüche definiert sind, ausgenommen 3-Amino-2-chlorcrotonsäure-(C₁-C₄)-alkylester.

10. Verwendung einer Verbindung der Formel I gemäß Anspruch 1 bei der Herstellung einer Verbindung der Formel IV, 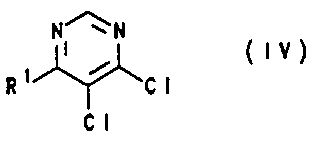 in welcher R¹ wie in einem der Ansprüche 1 und 3 bis 8 definiert ist.

## Claims

1. A process for preparing a compound of the formula I 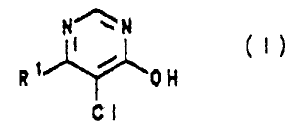 in which R¹ is alkyl, cycloalkyl, aryl or benzyl, all of which are optionally substituted, which comprises reacting a compound of the formula II 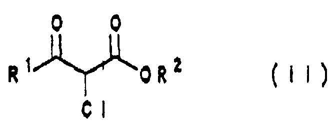 in which R¹ is as defined above, and R² is alkyl, benzyl or another carboxyl protective group, with ammonia or an ammonium salt to give a compound of the formula III 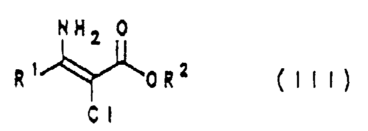 which is subsequently condensed in a polar protic solvent in the presence of a base with formamide to give a compound of the formula I.

2. The process as claimed in claim 1, wherein the intermediate of the formula III is not isolated.

3. The process as claimed in claim 1 or 2, wherein compounds of the formula I in which R¹ is (C₁-C₄)-alkyl, (C₁-C₄)-haloalkyl, (C₁-C₄)-alkoxy-(C₁-C₄)-alkyl or (C₁-C₄)-alkylthio-(C₁-C₄)-alkyl are prepared.

4. The process as claimed in any of claims 1 to 3, wherein compounds of the formula I in which R¹ is (C₁-C₄)-alkyl, preferably methyl or ethyl, or methoxymethyl are prepared.

5. The process as claimed in any of claims 1 to 4, wherein compounds of the formula I in which R¹ is ethyl are prepared.

6. The process as claimed in any of claims 1 to 5, which starts from compounds of the formula II in which R² is (C₁-C₄)-alkyl, benzyl or another carboxyl protective group.

7. The process as claimed in any of claims 1 to 6, which starts from compounds of the formula II in which R² is (C₁-C₄)-alkyl, preferably methyl, ethyl or tert-butyl, benzyl or modified benzyl.

8. The process as claimed in any of claims 1 to 7, which starts from compounds of the formula II in which R² is methyl.

9. A compound of the formula III as claimed in claim 1, in which R¹ and R² are as defined in any of the preceding claims, excepting (C₁-C₄)-alkyl 3-amino-2-chlorocrotonate.

10. The use of a compound of the formula I as claimed in claim 1 in the preparation of a compound of the formula IV 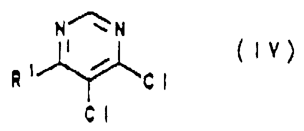 in which R¹ is as defined in any of claims 1 and 3 to 8.

## Revendications

1. Procédé pour préparer un composé de formule générale I 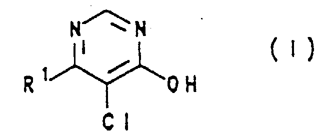 dans laquelle R¹ est un groupe alkyle, cycloalkyle, aryle ou benzyle, qui tous sont éventuellement substitués, caractérisé en ce qu'on fait réagir un composé de formule générale II 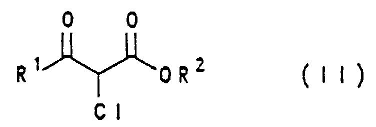 dans laquelle R¹ est tel que défini ci-dessus et R² est un groupe alkyle, benzyle ou un autre groupe protecteur de carboxyle, avec de l'ammoniac ou un sel d'ammonium pour donner un composé de formule générale III 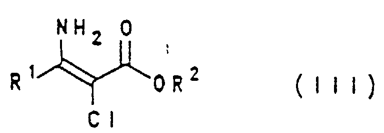 que l'on condense ensuite dans un solvant protique polaire en présence d'une base avec du formamide pour obtenir un composé de formule générale I.

2. Procédé selon la revendication 1, caractérisé en ce qu'on procède sans isolement du produit intermédiaire de formule III.

3. Procédé selon la revendication 1 ou 2, caractérisé en ce qu'on prépare des composés de formule I dans laquelle R¹ est un groupe alkyle en C₁-C₄, halogènalkyle en C₁-C₄, (alcoxy en C₁-C₄)-(alkyle en C₁-C₄) ou (alkylthio en C₁-C₄)-(alkyle en C₁-C₄).

4. Procédé selon l'une des revendications 1 à 3, caractérisé en ce qu'on prépare des composés de formule I dans laquelle R¹ est un groupe alkyle en C₁-C₄, de préférence méthyle ou éthyle,ou méthoxyméthyle.

5. Procédé selon l'une des revendications .1 à 4, caractérisé en ce qu'on prépare des composés de formule I dans laquelle R¹ est le groupe éthyle.

6. Procédé selon l'une des revendications 1 à 5, caractérisé en ce qu'on part de composés de formule II dans laquelle R² est un groupe alkyle en C₁-C₄, benzyle, ou un autre groupe protecteur de carboxyle.

7. Procédé selon l'une des revendications 1 à 6, caractérisé en ce qu'on part de composés de formule II dans laquelle R² est un groupe alkyle en C₁-C₄, de préférence méthyle, éthyle ou tert-butyle, benzyle ou benzyle modifié.

8. Procédé selon l'une des revendications 1 à 7, caractérisé en ce qu'on part de composés de formule II dans laquelle R² est le groupe méthyle.

9. Composé de formule III selon la revendication 1, dans lequel R¹ et R² sont tels que définis dans l'une des revendications précédentes, à l'exception des esters alkyliques en C₁-C₄ de l'acide 3-amino-2-chlorocrotonique.

10. Utilisation d'un composé de formule I selon la revendication 1 lors de la préparation d'un composé de formule IV 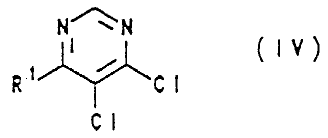 dans laquelle R¹ est tel que défini dans l'une des revendications 1 et 3 à 8.
